# EUROPÄISCHE PATENTANMELDUNG

(11) **EP 1 043 351 A2**
(43) Veröffentlichungstag der Anmeldung: **11.10.2000**
(21) Anmeldenummer: 00106935.0
(22) Anmeldetag: 31.03.2000
(51) Int. Cl.: C08G 18/67, C09D 175/04

(54) **Urethangruppenhaltige (Meth)acrylsäureester, deren Herstellung und Verwendung sowie diese umfassende strahlungshärtbare Überzugsmassen**

(30) Priorität: 01.04.1999 DE 19915070
(71) Anmelder: BASF AKTIENGESELLSCHAFT, 67056 Ludwigshafen (DE)
(72) Erfinder: Lokai, Matthias, 67677 Enkenbach-Alsenborn (DE); Beck, Erich, 68526 Ladenburg (DE); Reich, Wolfgang, 67133 Maxdorf (DE); Enenkel, Peter, 67258 Hessheim (DE); Marky, Herbert, 67659 Kaiserslautern (DE); Königer, Rainer, 67251 Freinsheim (DE)
(74) Vertreter: Kinzebach, Werner, Dr.

(57) **Zusammenfassung**

Die vorliegende Erfindung betrifft ein Verfahren zur Herstellung nichtdispergieraktiver urethangruppenhaltiger (Meth)acrylsäureester, wobei man
a) wenigstens eine hydroxylgruppenhaltige Verbindung mit (Meth)acrylsäure in einem Lösungsmittel unter Esterbildung umsetzt;
b) das Lösungsmittel und gegebenenfalls einen Teil nichtumgesetzter (Meth)acrylsäure entfernt;
c) das in Stufe a) oder b) resultierende Gemisch mit einer der Säurezahl des Gemisches entsprechenden Menge wenigstens einer epoxidgruppenhaltigen Verbindung umsetzt; und
d) das in Stufe c) resultierende Gemisch mit wenigstens einer isocyanatgruppenhaltigen Verbindung umsetzt,
sowie nach diesem Verfahren erhältliche nichtdispergieraktive urethangruppenhaltige (Meth)acrylsäureester, deren Verwendung in strahlungshärtbaren Überzugsmassen und diese Überzugsmassen.

## Beschreibung

Die vorliegende Erfindung betrifft nichtdispergieraktive urethangruppenhaltige (Meth)acrylsäureester, deren Herstellung und deren Verwendung in strahlungshärtbaren Überzugsmassen, sowie diese strahlungshärtbaren Überzugsmassen.

Strahlungshärtbare Bindemittel auf Basis von acrylgruppenhaltigen Polyestern sind bekannt. Insbesondere aufgrund ihrer lösungsmittelarmen oder sogar lösungsmittelfreien und schnellen Verarbeitbarkeit sind derartige Lackharze von großem Interesse. Wegen des geringen Lösungsgehalts dieser Systeme entfällt ein aufwendiges Ablüften und Aufarbeiten der Lösungsmittel. Die Emissionsgefahr durch Lösungsmittel wird wesentlich vermindert. Für eine wirtschaftliche Verarbeitbarkeit sind seitens der Bindemittel im Allgemeinen neben niedrigen Rohstoffkosten und einer hohen Reaktivität insbesondere auch ein geringer Bedarf an recht kostspieligen Reaktivverdünnern zur Einstellung geeigneter Verarbeitungsviskositäten von Bedeutung.

Eine Möglichkeit, den Zusatz von Reaktivverdünnern zu verringern oder ganz darauf zu verzichten, kann sich durch die Verwendung wässriger, strahlungshärtbarer Bindemitteldispersionen ergeben.

So beschreibt die DE-A-195 25 489 Polyesteracrylaturethan-Dispersionen auf Basis von hydroxylgruppenhaltigen Polyesteracrylatpräpolymeren. Die Herstellung dieser Dispersionen erfolgt durch die Addition von
A) 40 bis 90 Gew.-% eines oder mehrerer hydroxylgruppenhaltiger Polyesteracrylatpräpolymere mit einem OH-Gehalt von 40 bis 120 mg KOH/g und
B) 0,1 bis 20 Gew.-% einer oder mehrerer mono- und/oder difunktioneller gegenüber Isocyanatgruppen reaktiver Verbindungen, die kationische, anionische und/oder durch Ethereinheiten dispergierend wirkende Gruppen enthalten, mit
C) 10 bis 50 Gew.-% eines oder mehrerer Polyisocyanate sowie anschließender Umsetzung mit
D) 0,1 bis 10 Gew.-% eines oder mehrerer Di- und/oder Polyamine.

Derartige Dispersionen führen zu gehärteten Filmen, die in Bezug auf ihre mechanischen Eigenschaften, insbesondere einer guten Oberflächenhärte bei gleichzeitiger Flexibilität des Films, verbesserungswürdig sind. Ferner sind bei der Verwendung dieser Dispersionen als Beschichtungsmittel die mit dem Einsatz von Wasser zusammenhängenden geschilderten Nachteile zu berücksichtigen, so dass in vielen Fällen die Anwendung lösungsmittelarmer oder -freier Systeme zu bevorzugen ist.

Strahlungshärtbare Urethanpräparate werden in der DE 29 15 846 beschrieben.

In der EP-A-0 126 341 und der EP-A-0 127 766 werden Verfahren zur Herstellung strahlungshärtbarer (Meth)acrylsäureester beschrieben, wobei OH-Gruppen enthaltende Polyester mit überschüssiger Acrylsäure oder Methacrylsäure verestert werden und anschließend die restliche Acrylsäure oder Methacrylsäure durch eine Additionsreaktion mit Di- oder Polyglycidylethern zu nichtflüchtigen 2-Hydroxyacrylatestern umgesetzt wird.

Darauf aufbauend beschreibt schließlich die EP-B-0 279 303 strahlungshärtbare Acrylate, die erhältlich sind, indem man gleichzeitig
A) 1 Äquivalent eines zwei- bis sechswertigen, oxalkylierten C₂-C₁₀-Alkohols mit
B) 0,05 bis 1 Äquivalent einer zwei- bis vierwertigen C₃-C₃₆-Carbonsäure oder deren Anhydrid und
C) 0,1 bis 1,5 Äquivalente Acrylsäure und/oder Methacrylsäure
umsetzt, und weiterhin die überschüssigen Carboxylgruppen mit der äquivalenten Menge einer Epoxidverbindung umsetzt. Werden diese Beschichtungs- und Überzugsmittel durch Elektronenstrahlen oder nach Zusatz von Photoinitiatoren durch UV-Strahlen vernetzt, ergeben sich Filme, die den Anforderungen der Praxis in der Regel gerecht werden. Nichtsdestotrotz kann es in einigen Fällen wünschenswert sein, Härte, Flexibilität und/oder chemische Beständigkeit der resultierenden Filme zu verbessern.

Der vorliegenden Erfindung liegt daher die Aufgabe zugrunde, Beschichtungs- und Überzugsmittel auf Basis von (Meth)acrylsäureestern zur Verfügung zu stellen, mit denen strahlungsgehärtete Verfilmungen mit hoher Härte, guter Flexibilität und ausreichender chemischer Beständigkeit erhalten werden können.

Dies gelingt überraschenderweise mit nichtdispergieraktiven urethangruppenhaltigen (Meth)acrylsäureestern, die erhältlich sind, indem man
a) wenigstens eine hydroxylgruppenhaltige Verbindung mit (Meth)acrylsäure in einem Lösungsmittel unter Esterbildung umsetzt;
b) das Lösungsmittel und gegebenenfalls einen Teil nicht umgesetzter (Meth)acrylsäure entfernt;
c) das in Stufe a) oder b) resultierende Gemisch mit einer der Säurezahl des Gemisches entsprechenden Menge wenigstens einer epoxidgruppenhaltigen Verbindung umsetzt; und
d) das in Stufe c) resultierende Gemisch mit wenigstens einer isocyanatgruppenhaltigen Verbindung umsetzt.

Die erfindungsgemäßen urethangruppenhaltigen (Meth)acrylsäureester sind nichtdispergieraktiv, d.h. sie bilden ohne den Zusatz weiterer Hilfsstoffe keine stabilen Dispersionen und/oder Emulsionen aus. Die erfindungsgemäßen urethangruppenhaltigen (Meth)acrylsäureester weisen nicht ausreichend dispergieraktive Gruppen auf, um stabile Dispersionen und/oder Emulsionen auszubilden. Unter dispergieraktiven Gruppen versteht man in der Regel polare funktionelle Gruppen, wie ionogene und/oder ionische Gruppen, insbesondere Carbonsäuregruppen, Sulfonsäuregruppen, Phosphonsäuregruppen, Phosphorsäuregruppen, Alkalimetall- und Ammoniumsalze davon, quaternäre Ammoniumgruppen sowie Ethergruppen. Hydroxycarbonsäuren, Aminosäuren, Aminosulfonsäuren sowie Polyetherole oder α,ω-Diaminopolyether mit Molekulargewichten von etwa 500 bis 2000 werden üblicherweise zum Aufbau dispergieraktiver Polyurethane verwendet, die dann in Form von Dispersionen verarbeitet werden. Die Herstellung der erfindungsgemäßen urethangruppenhaltigen (Meth)acrylsäureester erfolgt vorzugsweise ohne die vorstehend genannten dispergieraktiven Komponenten; insbesondere werden keine dispergieraktiven Gruppen über die Umsetzung mit isocyanatgruppenhaltigen Verbindungen in die urethangruppenhaltige (Meth)acrylsäureester eingebaut.

Als hydroxylgruppenhaltige Verbindungen mit zwei oder mehreren Hydroxylgruppen pro Molekül eignen sich beispielsweise zwei- bis sechswertige C₂-C₂₀-Polyole, vorzugsweise C₂-C₁₀-Polyole, beispielsweise Diole, wie Ethylenglykol, 1,2-Butandiol, 2-Methyl-1,3-propandiol, 1,4-Butandiol, 1,5-Pentandiol, Neopentylglykol, 1,6-Hexandiol, 2-Methylpentan-1,5-diol, 2-Ethylbutan-1,4-diol, 1,10-Decandiol, Diethylenglykol, 2,2,4-Trimethylpentan-1,5-diol, 2,2-Dimethylpropan-1,3-diol, 1,4-Dimethylolcyclohexan, 1,6-Dimethylolcyclohexan, 1,1'-Isopropylen-bis-(p-phenylenoxy)-di-β-ethanol, 2,2-Bis-(4-hydroxyphenyl)propan (Bisphenol A); Triole, wie Glycerin, Trimethylolethan, Trimethylolpropan, Trimethylolbutan; Tetraole, wie Pentaerythrit, Ditrimethylolpropan; Hexole, wie Erythrit, Sorbit und Dipentaerythrit.

Bevorzugt sind drei- bis sechswertige C₃-C₆-Alkohole, wie Trimethylolpropan, Glycerin, Pentaerythrit und Sorbit.

Geeignet sind auch Oxalkylierungsprodukte. Unter Oxalkylierungsprodukten werden erfindungsgemäß die aus der Umsetzung hydroxylgruppenhaltiger Verbindungen mit Alkylenoxiden oder Alkylenoxidgemischen, wie Ethylenoxid, Propylenoxid, Tetrahydrofuran und/oder Butylenoxid, nach üblichen Verfahren erhältlichen Polymerisationsprodukte verstanden. Bevorzugt sind Oxethylierungs- und Oxpropylierungsprodukte der vorstehend genannten Polyole. Der Oxalkylierungsgrad dieser Polyetherpolyole liegt in der Regel zwischen 1 und 30, bevorzugt zwischen 1 und 10.

Bevorzugt ist oxalkyliertes, insbesondere ethoxyliertes Trimethylolpropan.

Die vorstehend genannten hydroxylgruppenhaltigen Verbindungen mit zwei oder mehreren Hydroxylgruppen pro Molekül weisen (mittlere) Molekulargewichte auf, die im Bereich von vorzugweise 62 bis 4000, insbesondere bei 80 bis 800 und vor allem bei 90 bis 500 liegen.

Als hydroxylgruppenhaltige Verbindungen mit einer Hydroxylgruppe pro Molekül eignen sich C₅-C₃₀-Monoalkohole, vorzugsweise C₈-C₂₀-Monoalkohole, beispielsweise 2-Ethylhexanol, Laurylalkohol, Stearylalkohol, 4-t-Butylcyclohexanol, 3,3,5-Trimethylcyclohexanol, 2-Methyl-3-phenylpropan-1-ol und Phenylglykol.

Geeignet sind auch entsprechende Oxalkylierungsprodukte, vorzugsweise Oxethylierungs- und Oxpropylierungsprodukte von C₂-C₁₂-Monoalkoholen. Der Oxalkylierungsgrad liegt in der Regel zwischen 1 bis 10, vorzugsweise zwischen 1 bis 5.

Die vorstehend genannten hydroxylgruppenhaltigen Verbindungen mit einer Hydroxylgruppe pro Molekül weisen vorzugsweise Molekulargewichte zwischen 130 und 300 auf.

Als hydroxylgruppenhaltige Verbindungen eignen sich auch Polyesterpolyole aus zwei- bis sechswertigen, gegebenenfalls oxalkylierten C₂-C₂₀-Polyolen und zwei- bis vierwertigen C₃-C₃₆-Carbonsäuren oder veresterbaren Derivaten davon. Derartige Polyesterpolyole können in einem separaten Veresterungsschritt hergestellt und dann in der erfindungsgemäßen Stufe a) eingesetzt werden. Sie können aber auch in situ während der in Stufe a) durchgeführten Acrylsäure- oder Methacrylsäureveresterung hergestellt werden, indem man in Stufe a) gleichzeitig zwei- bis sechswertige, gegebenenfalls oxalkylierte C₂-C₂₀-Polyole mit zwei- bis vierwertigen C₃-C₃₆-Carbonsäuren oder veresterbaren Derivaten davon und (Meth)acrylsäure umsetzt. Hierzu eignen sich vornehmlich die zuvor beschriebenen zwei- bis sechswertigen, gegebenfalls oxalkylierten C₂-C₁₀-Polyole und insbesondere deren bevorzugte Vertreter.

Erfindungsgemäß geeignet sind auch Polylactonpolyole aus Lactonen, d.h. cyclischen Hydroxy-C₅-C₁₀-Alkylcarbonsäuren, mit vorzugsweise 6- bis 8-gliedrigen Ringen, insbesondere Caprolactonen und vor allem ε-Caprolacton, und Polyolen, wie den oben genannten, gegebenenfalls oxalkylierten Polyolen, wobei Diole und Triole bevorzugt sind. Derartige Polylactonpolyole können wie die vorstehend genannten Polyesterpolyole nach bekannten Verfahren hergestellt werden.

Geeignete zwei- bis vierwertige C₃-C₃₆-Carbonsäuren sind insbesondere C₄-C₁₅-Dicarbonsäuren sowie veresterbare Derivate davon, wie Anhydride oder C₁-C₄-Alkylester, beispielsweise Bernsteinsäure, Bernsteinsäureanhydrid, Glutarsäure, Glutarsäureanhydrid, Adipinsäure, Pimelinsäure, Korksäure, Sebacinsäure, Dodecandisäure, Phthalsäure, Phthalsäureanhydrid, Terephthalsäure, Isophthalsäure, Maleinsäure, Maleinsäureanhydrid, Fumarsäure, Citraconsäure, Tetrahydrophthalsäure, Tetrahydrophthalsäureanhydrid, Hexahydrophthalsäure, Hexachlorendomethylentetrahydrophthalsäure, dimere Linolsäure, Trimellithsäure, Trimellithsäureanhydrid, Pyromellithsäure, Pyromellithsäureanhydrid, Citronensäure und Weinsäure, Isomere und Hydrierungsprodukte der vorstehend genannten Carbonsäuren und deren veresterbare Derivate, sowie Gemische davon. Bevorzugt sind Adipinsäure, Phthalsäure, Phthalsäureanhydrid, Maleinsäureanhydrid und Fumarsäure.

Bevorzugte urethangruppenhaltige (Meth)acrylsäureester sind erhältlich, indem man in Stufe a) in Bezug auf die Hydroxylgruppen der hydroxylgruppenhaltigen Verbindung(en) einen Überschuss an (Meth)acrylsäure einsetzt, vorzugsweise bis zu einem Äquivalentverhältnis von 1:1,5 und insbesondere in einem Äquivalentverhältnis von 1:1,1 bis 1:1,25. Setzt man in Stufe a) gleichzeitig Polyol, Carbonsäure und (Meth)acrylsäure um, so sind Äquivalentverhältnisse von Polyol:Carbonsäure: (Meth)acrylsäure von 1:0,05-1:0,1-1,5 und insbesondere von 1:0,1-0,6:0,5-0,9 bevorzugt.

Die Veresterung in Stufe a) erfolgt vorzugsweise in einem Lösungsmittel, das mit Wasser ein azeotropes Gemisch bildet. So kann das unter Esterbildung entstehende Wasser während der Umsetzung azeotrop entfernt werden. Vorteilhaft sind Lösungsmittel, die zwar mit Wasser, nicht aber mit den übrigen Reaktionsedukten und -produkten ein Azeotrop, insbesondere ein Minimumazeotrop, bilden. Es eignen sich beispielsweise aliphatische, cycloaliphatische oder aromatische Kohlenwasserstoffe sowie Gemische davon. Bevorzugt sind Kohlenwasserstoffe oder Kohlenwasserstoffgemische mit einem Siedebereich von 50 bis 130 °C und insbesondere zwischen 60 und 110 °C. Zu nennen sind beispielsweise Alkane, wie n-Hexan, n-Heptan sowie deren Isomere und Gemische davon, Cycloalkane, wie Cyclohexan oder Methylcyclohexan, aromatische Kohlenwasserstoffe, wie Benzol, Toluol und Xylolisomere. Besonders bevorzugte Schleppmittel sind Heptan, Cyclohexan, Methylcyclohexan und Toluol sowie Hexan, Heptan und deren Isomere sowie Gemische davon mit einem Siedebereich zwischen 60 und 130 °C.

Die Menge des zugesetzten Kohlenwasserstoffes oder Kohlenwasserstoffgemisches kann je nach verwendeter Apparatur zwischen der 0,1- und 2-fachen Menge des Reaktionsgemisches aus hydroxylgruppenhaltiger Verbindung und (Meth)acrylsäure variieren. Besonders vorteilhaft ist ein Verhältnis Reaktionsgemisch zu Kohlenwasserstoff von 1:0,20 bis 1:0,8.

Die während der Veresterungsreaktion angelegte Temperatur wird vorzugsweise so gewählt, dass azeotrope Bedingungen herrschen. Unter derartigen Bedingungen wirkt das Lösungsmittel als Schleppmittel zur Entfernung des entstehenden Reaktionswassers. Die Veresterung erfolgt in der Regel bei erhöhter Temperatur, im Allgemeinen bei 40 bis 130 °C, vorzugsweise bei 70 bis 120 °C.

Bevorzugte urethangruppenhaltige (Meth)acrylsäureester sind erhältlich, wenn man die Umsetzung in Stufe a) in Gegenwart wenigstens eines Polymerisationsinhibitors durchführt. Zur Vermeidung einer vorzeitigen Polymerisation reicht es im Allgemeinen aus, geringe Mengen an Inhibitor(en) zuzusetzen. Dabei kann es sich um die üblichen, zur Verhinderung einer thermischen Polymerisation verwendeten Verbindungen handeln. Geeignet sind beispielsweise Inhibitoren auf Basis ein- und mehrwertiger Phenole, wie p-Methoxyphenol, 2,6-Di-t-butylphenole, insbesondere 2,6-Di-t-butyl-p-cresol, Chinone, wie p-Benzochinon, Hydrochinone, Hydrochinonmono-C₁-C₄-alkylether, insbesondere Hydrochinonmonomethylether, Resorcinmonomethylether, Thiodiphenylamine, auch Phenothiazine genannt, 2,2,6,6-Tetramethylpiperidin-1-yloxy, 4-Hydroxy-2,2,6,6-Tetramethylpiperidin-1-yloxy, Phosphorigsäureester, und Zinn-II-Verbindungen. Vorteilhaft sind Kombinationen aus obigen Phenolverbindungen mit Phenothiazin oder Zinn-II-Verbindungen sowie die Kombination aus Zinn-II-Verbindungen mit Phenolverbindungen und Phenothiazin. Die Gesamtmenge an Polymerisationsinhibitor beträgt im Allgemeinen 0,001 bis 2,0 Gew.-%, vorzugsweise 0,005 bis 0,5 Gew.-%, bezogen auf die Menge an Veresterungsgemisch aus hydroxylgruppenhaltiger Verbindung bzw. hydroxylgruppenhaltigen Verbindungen und (Meth)acrylsäure. Die Verbindungen auf Phenolbasis werden vorzugsweise in Mengen von 0,5 bis 1 Gew.-%, die Zinn-II-Verbindungen in Mengen von 0,01 bis 1 Gew.-% Sn und Phenothiazin in Mengen von 0,001 bis 0,1 Gew.-%, jeweils bezogen auf die Menge an Veresterungsgemisch, eingesetzt. In der Regel führen die vorstehend genannten Polymerisationsinhibitoren zu urethangruppenhaltigen (Meth)acrylsäureestern mit geringen Farbzahlen. Da farblose Produkte bevorzugt sind, kann der Einsatz geeigneter, die Produktverfärbung verringernder Additive, wie Triphenylphosphit und hypophsphoriger Säure, insbesondere dann von Vorteil sein, wenn die Temperaturbelastung während des Herstellungsverfahrens zu einer wenn auch nur leichten Verfärbung führt.

Im Übrigen erfolgt die Veresterung nach allgemein bekannten Methoden in Gegenwart von sauren Veresterungskatalysatoren, wie anorganischen oder organischen Säuren oder sauren Ionenaustauschern, wobei Schwefelsäure und Sulfonsäuren, wie p-Toluolsulfonsäure und Methansulfonsäure, bevorzugt sind. Die sauren Veresterungskatalysatoren werden im Allgemeinen in Mengen von 0,1 bis 3 Gew.-%, bezogen auf das Veresterungsgemisch, eingesetzt.

Die Veresterung wird im Allgemeinen bis zu einem Umsatz von mindestens 85 %, vorzugsweise von 90 bis 95 %, bezogen auf eingesetzte Menge an Hydroxylgruppen geführt. Der Veresterungsgrad kann anhand der ausgekreisten Wassermenge bestimmt werden.

Das Lösungsmittel wird in Stufe b) entfernt. Dies geschieht vorzugsweise durch Destillation, und zwar entweder im Anschluß an die Stufe a), d.h. nach beendeter Auskreisung des Reaktionswassers, oder gegen Ende der Stufe c), beispielsweise dann, wenn die Säurezahl des Reaktionsgemisches auf 15 bis 5 mg KOH/g gefallen ist. Durch die Entfernung des Lösungsmittels wird gegebenenfalls ein Teil nicht umgesetzter (Meth)acrylsäure mitentfernt. Je nach der anfänglich eingesetzten (Meth)acrylsäuremenge, der Reaktionsführung, dem Reaktionsfortschritt und/oder der für die Stufe c) erwünschten (Meth)acrylsäuremenge kann auch ein Teil der nicht umgesetzten (Meth)acrylsäure gezielt destillativ entfernt werden, indem man geeignete Destillationsbedingungen wählt. Die Entfernung des Lösungsmittels und gegebenenfalls eines Teils nicht umgesetzter (Meth)acrylsäure erfolgt vorzugsweise durch Vakuumdestillation.

In Stufe b) kann auch die vorzugsweise vollständige Neutralisation des Veresterungskatalysators mit einer äquivalenten Menge einer Base erfolgen. Als Base können Alkalimetallbasen, wie Natronlauge, Kalilauge, Natriumcarbonat, Natriumhydrogencarbonat, Kaliumcarbonat oder Kaliumhydrogencarbonat, und Erdalkalimetallbasen, wie Calciumhydroxid, Calciumoxid, Magnesiumhydroxid oder Magnesiumcarbonat, sowie Ammoniak und Amine, wie Trimethylamin, Triethylamin, Triisopropylamin etc. verwendet werden.

Bevorzugte urethangruppenhaltige (Meth)acrylsäureester sind erhältlich, wenn man in Stufe b) die Entfernung des Lösungsmittels und gegebenenfalls eines Teils nichtumgesetzter (Meth)Acrylsäure so führt, dass die Säurezahl des resultierenden Gemisches 200 bis 5 mg KOH/g, vorzugsweise 80 bis 15 mg KOH/g beträgt. Säurezahlen sind in an sich bekannter Art und Weise bestimmbar.

Ein wesentlicher Vorteil des erfindungsgemäßen Veresterungsverfahrens besteht darin, dass auf Waschvorgänge zur Entfernung des Veresterungskatalysators und nichtumgesetzter (Meth) acrylsäure verzichtet werden kann. In diesem Fall dient der Destillationsschritt zur Abtrennung des Lösungsmittels, wobei gegebenenfalls ein Teil nichtumgesetzter (Meth)acrylsäure mitentfernt werden kann.

Das in Stufe b) resultierende Gemisch wird dann in Stufe c) mit einer der Säurezahl des Gemisches entsprechenden Menge wenigstens einer epoxidgruppenhaltigen Verbindung umgesetzt. Die Säurezahl ergibt sich im Wesentlichen durch Carbonsäuregruppen nicht-umgesetzter (Meth)acrylsäure und gegebenenfalls weiterer in Stufe a) eingesetzter Carbonsäuren bzw. erzeugter hydroxylgruppenhaltiger Carbonsäuren.

Als epoxidgruppenhaltige Verbindungen sind sowohl Monoepoxidverbindungen als auch polyfunktionelle Epoxide, insbesondere di- oder trifunktionelle Epoxide, geeignet. Zu nennen sind beispielsweise epoxidierte Olefine, Glycidylester von gesättigten oder ungesättigten Carbonsäuren oder Glycidylether aliphatischer oder aromatischer Polyole. Als Monoepoxidverbindung insbesondere geeignet sind beispielsweise Versaticsäureglycidylester und Alkyl- bzw. Arylglycidylether, wie n-Butylglycidylether, 2-Ethylhexylglycidylether, Phenylglycidylether, o-Kresylglycidylether oder vorzugsweise 1,2-Epoxybutan. Besonders geeignete polyfunktionelle Epoxide sind di- oder trifunktionelle Glycidylether oder Glycidylester. Bevorzugt sind Polyglycidylverbindungen vom Bisphenol-A-Typ oder Glycidylether mehrfunktioneller Alkohole, wie Butandiol, Glycerin oder Pentaerythrit. Insbesondere bevorzugt sind Bisphenol-A-diglycidylether, beispielsweise Epikote® 828, Butandioldiglycidylether und Pentaerythrittriglycidylether.

Bevorzugte urethangruppenhaltige (Meth)acrylsäureester sind erhältlich, wenn man für den Fall, dass hydroxylgruppenhaltige Verbindungen mit nur einer Hydroxylgruppe pro Molekül mit (Meth)acrylsäure verestert werden, als epoxidgruppenhaltige Verbindungen Monoepoxidverbindungen, und für den Fall, dass hydroxylgruppenhaltige Verbindungen mit zwei oder mehr Hydroxylgruppen pro Molekül mit (Meth)acrylsäure verestert werden, als epoxidgruppenhaltige Verbindungen solche mit mindestens zwei Epoxidgruppen pro Molekül einsetzt.

Die Umsetzung in Stufe c) erfolgt in der Regel bei erhöhter Temperatur, vorzugsweise bei 80 bis 130 °C und insbesondere bei 95 bis 115 °C. Im Hinblick auf eine effektive Umsetzung von Carboxylmit Epoxidgruppen kann es von Vorteil sein, die Umsetzung in Gegenwart geeigneter Katalysatoren durchzuführen. Zu nennen sind hier insbesondere tertiäre Amine, wie Tri-C₁-C₄-amine, beispielsweise Tributylamin, quaternäre Ammoniumverbindungen, wie Tetra-C₁-C₄-alkylammoniumhalogenide, beispielsweise Tetrabutylammoniumbromid, Phosphine oder Lewisbasen, beispielsweise Thiodiglykole, insbesondere Thiodiglykol selbst.

Bevorzugte urethangruppenhaltige (Meth)acrylsäureester sind erhältlich, wenn man in Stufe c) die Umsetzung mit epoxidgruppenhaltigen Verbindungen so weit führt, dass die Säurezahl des resultierenden Gemisches höchstens 10 mg KOH/g, vorzugsweise höchstens 5 KOH/g, beträgt.

Weitere bevorzugte urethangruppenhaltige (Meth) acrylsäureester sind erhältlich, wenn das in Stufe c) resultierende Gemisch eine Hydroxylzahl in einem Bereich von 25 bis 300 mg KOH/g und insbesondere von 40 bis 150 mg KOH/g aufweist. Hydroxylzahlen sind in an sich bekannter Weise bestimmbar.

Ferner sind bevorzugte urethangruppenhaltige (Meth)acrylsäureester erhältlich, wenn das in Stufe c) resultierende Gemisch Viskositäten von 0,5 bis 20 Pas, vorzugsweise von 1 bis 15 Pas, aufweist.

Das in Stufe c) resultierende (Meth)acrylsäureestergemisch wird dann in Stufe d) mit wenigstens einer isocyanatgruppenhaltigen Verbindung umgesetzt. Als isocyanatgruppenhaltige Verbindungen eignen sich Mono- und vorzugsweise Polyisocyanate mit zwei oder mehreren Isocyanatgruppen pro Molekül. Erfindungsgemäß bevorzugt sind Polyisocyanate mit 2 bis 5 Isocyanatgruppen pro Molekül, beispielsweise aliphatische, cycloaliphatische und aromatische Di-, Tri- und Polyisocyanate. Geeignete Diisocyanate sind z. B. Tetramethylendiisocyanat, Hexamethylendiisocyanat und dessen Trimere, 2,3,3-Tetramethylhexamethylendiisocyanat, 1,4-Cyclohexylendiisocyanat, Dicyclohexylmethandiisocyanat, Isophorondiisocyanat, 1,4-Phenylendiisocyanat, 2,4- und 2,6-Toluylendiisocyanat, sowie Isomerengemische davon (beispielsweise 80 % 2,4- und 20 % 2,6-Isomer), Xylendiisocyanat, Tetramethylxylylendiisocyanat, 1,5-Naphthylendiisocyanat, 2,4- und 4,4'-Diphenylmethandiisocyanat. Ein geeignetes Triisocyanat ist beispielsweise Triphenylmethan-4,4',4''-triisocyanat. Weiterhin geeignet sind Polyisocyanate, die durch Addition der zuvor genannten Isocyanate an polyfunktionelle Hydroxyl- oder Amingruppen enthaltende Verbindungen erhältlich sind. Geeignet sind auch Polyisocyanate, die durch Allophanat-, Biuret- oder Isocyanuratbildung entstehen. Hierzu gehört das Isocyanurat des Hexamethylendiisocyanats. Weiterhin geeignet sind blockierte, reversibel verkappte Polykisisocyanate, wie 1,3,5-Tris-[6-(1-methylpropylidenaminoxycarbonylamino)hexyl]-2,4,6-trioxohexahydro-1,3,5-triazin. Besonders bevorzugt sind Hexamethylendiisocyanat und Isophorondiisocyanat und deren Gemische sowie das Isocyanurat des Hexamethylendiisocyanats.

Das Molmengenverhältnis von Isocyanatgruppen der in Stufe d) umgesetzten isocyanatgruppenhaltigen Verbindungen zu Hydroxylgruppen des in Stufe c)resultierenden Gemisches sollte weniger als 1 betragen und liegt im Allgemeinen in einem Bereich von etwa 0,1 bis 0,75, bevorzugt bei etwa 0,15 bis 0,50.

Es kann vorteilhaft sein, die Umsetzung in Stufe d) zu beschleunigen, indem man bekannte Katalysatoren für Isocyanatadditionen zusetzt. Zu nennen sind hier beispielsweise Triethylamin, 1,4-Diazabicyclo-[2,2,2]-oktan, Zinndioctoat oder Dibutylzinndilaurat.

Besonders bevorzugte urethangruppenhaltige (Meth)acrylsäureester sind erhältlich, indem man
a) ethoxyliertes Trimethylolpropan und Adipinsäure mit einem Überschuss an (Meth)acrylsäure in einem mit Wasser ein azeotropes Gemisch bildenden Kohlenwasserstoff, vorzugsweise Methylcyclohexan, unter Esterbildung umsetzt;
b) den Kohlenwasserstoff und einen Teil nichtumgesetzter (Meth)acrylsäure destillativ, vorzugsweise unter Vakuum, entfernt;
c) das in Stufe b) resultierende Gemisch mit einer der Säurezahl des Gemisches entsprechenden Menge an Bisphenol-A-diglycidylether umsetzt; und
d) das in Stufe c) resultierende Gemisch mit einem Gemisch aus Hexamethylendiisocyanat und dessen Isocyanurat umsetzt.

Gegenstand der vorliegenden Erfindung sind die vorstehend beschriebenen Verfahren sowie nichtdispergieraktive urethangruppenhaltige (Meth)acrylsäureester, die nach einem dieser Verfahren erhältlich sind.

Ein weiterer Gegenstand der vorliegenden Erfindung ist die Verwendung dieser nichtdispergieraktiven urethangruppenhaltigen (Meth)acrylsäureester in strahlungshärtbaren Überzugsmassen. Derartige Überzugsmassen eignen sich vorzugsweise zur Herstellung von Beschichtungen, z. B. von flexiblen und gegebenenfalls saugfähigen Substraten, wie Holz und Holzwerkstoffen, beispielsweise Papier und Karton, sowie Leder, oder von nichtflexiblen Substraten aus Metall oder Kunststoff. Vorzugsweise eignen sie sich zur Herstellung von hochwertigen, kratzfesten und chemikalienresistenten Lacküberzügen.

Die Vernetzung, d. h. die Härtung der erfindungsgemäßen Polymerisate kann mit energiereicher Strahlung, wie UV-, Elektronen-, Röntgen- oder γ-Strahlung, erfolgen. Dabei ist die UV-Härtung besonders bevorzugt. Sie kann gewünschtenfalls in Gegenwart üblicher Photoinitiatoren, wie z. B. aromatischer Ketonverbindungen, wie Benzophenon, Alkylbenzophenonen, Michler's Keton, Anthron, halogenierten Benzophenonen, 2,4,6-Trimethylbenzoyldiphenylphosphinoxid, Phenylglyoxylsäureestern, Anthrachinon und dessen Derivaten, Benzoinethern, Benzilketalen, Hydroxyalkylphenonen, Acylphosphinoxiden oder Acetophenon-Derivaten erfolgen. Dabei können auch Gemische dieser Verbindungen eingesetzt werden. Die Einsatzmenge der Photoinitiatoren beträgt im Allgemeinen etwa 0,01 bis 20 Gew.-%, bevorzugt 0,1 bis 10 Gew.-% und insbesondere 0,5 bis 4 Gew.-%, bezogen auf das Gesamtgewicht der zu härtenden Komponenten. Geeignete Strahlungsquellen sind z. B. Mitteldruckquecksilberdampfstrahler, wie sie etwa auch in "UV Curing: Science and Technology" (Hrsg. S. P. Pappas) beschrieben werden. Erfolgt die Härtung mittels Elektronenstrahlen, so werden im Allgemeinen Beschleunigungsspannungen von 150 bis 500 kV verwendet.

Gegebenenfalls können die erfindungsgemäßen urethangruppenhaltigen (Meth)acrylsäureester zur Verarbeitung mit weiteren aus der Strahlungshärtung bekannten Reaktivverdünnern versetzt werden. Bei den Reaktivverdünnern handelt es sich im Allgemeinen um copolymerisierbare Monomere, wie (Meth)acrylsäurealkylester oder den entsprechenden Estern der Malein-, Fumar-, Tetrahydrophthal-, Croton-, Isocroton-, Vinylessig- und Itaconsäure. Auch Monomere mit mehr als einer Doppelbindung pro Molekül sind geeignet, wie die Di-, Tri- und Tetra(meth)acrylate von Diolen, Triolen bzw. Tetraolen, sowie Acrylate von alkoxylierten Diolen und Triolen, beispielsweise Ethylenglykol, Diethylenglykol, Propylenglykol, Trimethylenglykol, Neopentylglykol, 1,2-Butandiol, 1,3-Butandiol, 1,6-Hexamethylenglykol, 1,10-Deacamethylenglykol, Trimethylolpropan, Pentaerythrit. Beispielhaft seien hier lediglich genannt 4-t-Butylcyclohexylacrylat, Phenoxyethylacrylat, Hexandioldiacrylat, Tripropylenglykoldiacrylat, Trimethylolpropantriacrylat.

Zu weiteren gegebenenfalls geeigneten Additiven gehören Synergisten, wie Amine, Thioverbindungen mit α-endständigen C-H-Gruppen sowie Ether; nichtmitvernetzende Lösungsmittel, wie aromatische Kohlenwasserstoffe oder Ester, beispielsweise Butylacetat; Extender, wie Talkum, Schwerspat oder Silikate; Entschäumer; Verlaufsmittel; filmbildende Hilfsmittel, wie Cellulose-Derivate.

Somit sind Gegenstand der voliegenden Erfindung auch strahlungshärtbare Überzugsmassen, die wenigstens einen erfindungsgemäßen urethangruppenhaltigen (Meth)acrylsäureester umfassen und zusätzlich Photoinitiatoren und weitere, insbesondere die vorstehend beschriebenen Additive enthalten können.

Die erfindungsgemäßen überzugsmassen können mittels Spritzen, Tauchen, Rakeln, Streichen, Walzen, Fluten oder ähnlicher Maßnahmen auf die zu behandelnden Flächen appliziert werden. Die Filme werden dann durch Strahlung in an sich bekannter Weise gehärtet.

Die erfindungsgemäß erhaltenen gehärteten Filme werden den Anforderungen der Praxis voll gerecht. Insbesondere zeichnen sie sich durch eine Härte, Flexibilität und chemische Beständigkeit aus, die mit den entsprechenden herkömmlichen (Meth)acrylsäureestern nicht erreicht wird.

Die vorliegende Erfindung wird durch die nachfolgenden Beispiele erläutert, ohne darauf beschränkt zu sein.

### Beispiel 1: Urethangruppenhaltiger Polyesteracrylatester

20,9 kg ethoxyliertes Trimethylolpropan (OH-Zahl 610 mg KOH/g), 5,72 kg Adipinsäure und 14,1 kg Acrylsäure wurden zu 17,6 l Methylcyclohexan gegeben. Weiterhin wurden 0,2 kg Schwefelsäure, 0,12 kg Hydrochinonmonomethylether, 0,041 kg 2,6-Di-t-butyl-p-kresol, 0,041 kg Triphenylphosphit, 0,041 kg hypophosphorige Säure und 0,004 kg Phenothiazin zugegeben. Diese Mischung wurde auf Rückfluss erhitzt und das Veresterungsvasser über einen Dean-Stark-Wasserabscheider entfernt. Nachdem die Veresterung beendet war, wurde das Schleppmittel und ein Teil der überschüssigen Acrylsäure unter Vakuum abdestilliert, bis die Säurezahl bei 37,8 mg KOH/g lag. Das Vakuum wurde gebrochen und 0,88 kg Tetrabutylammoniumbromid und 4,3 kg Epikote® 828 wurden zugegeben. Das Reaktionsgemisch wurde bei 107 °C 5,5 Stunden gerührt, bis die Säurezahl auf 4,1 mg KOH/g gefallen war. Daraufhin wurde das Produkt auf 70 °C abgekühlt, und es wurden 3,3 kg Basonat HI 100 innerhalb von 10 Minuten zudosiert. Die Temperatur des Reaktionsgemisches wurde auf 80 °C angehoben und 4 Stunden gehalten. Dann wurde das resultierende Reaktionsgemisch abgefüllt. Das Produkt wies eine Viskosität von 21,2 Pas auf.

### Beispiel 2: Herkömmlicher Polyesteracrylatester

Es wurde exakt wie in Beispiel 1 verfahren bis die Säurezahl auf 4,1 mg KOH/g gefallen war. Das resultierende Reaktionsgemisch wurde auf 80 °C abgekühlt und abgefüllt. Das Produkt wies eine Viskosität von 3,5 Pas auf.

### Beispiel 3: UV-Härtung

100 Teile urethangruppenhaltiger Acrylsäureester gemäß Beispiel 1 wurden mit 3 Teilen Photoinitiatorengemisch und 8,5 Teilen Lösungsmittel vermischt. Zum Vergleich wurden 100 Teile Polyesteracrylat gemäß Beispiel 2 mit 3 Teilen Photoinitiatorengemisch vermischt. Die verwendeten Komponenten sowie einige physikalische Eigenschaften der resultierenden Gemische sind in folgender Tabelle 1 angegeben:

**Tabelle 1**

| Komponenten | | 1 | 2 | 3 | 4 |
|---|---|---|---|---|---|
| Polyester gemäß Bsp. 1 | | 100 | | 100 | |
| Polyester gemäß Bsp. 2 | | | 100 | | 100 |
| Irgacure 184 | | 3 | 3 | | |
| Irgacure 500 | | | | 3 | 3 |
| Butylacetat | | 8,5 | | 8,5 | |
| | | | | | |

| Physikalische Tests | Bedingungen | 1 | 2 | 3 | 4 |
|---|---|---|---|---|---|
| Viskosität Pas | 23°C | 4 | 4,5 | 4 | 3,2 |
| Reaktivität [m/min] | 8 g/m² | 11 | 5 | 23 | 5 |
| Reaktivität [m/min] | 50 g/m² | 11 | 5 | 23 | 5 |

Die Härtung erfolgte mit einem Strahler der Firma IST bei 120 W/cm.

### Beispiel 4: Prüfung der Lackeigenschaften

Die gemäß Beispiel 3 erhaltenen Filme wurden einer anwendungstechnischer Prüfung unterzogen. Es wurden die in Tabelle 2 angegebenen Resultate erzielt:

**Tabelle 2**

| Testbedingungen | Physikalische Tests | 1 | 2 | 3 | 4 |
|---|---|---|---|---|---|
| 100 g/m² | Pendeldämpfung [Schläge] | 35 | 26 | 27 | 22 |
| 25 g/m² | Erichsen-Tiefung [mm] | 4,8 | 5,1 | 5 | 5 |
| 25 g/m² | YI (unbelichtet 1x10 m/ min) | 3,6 | 3,7 | 3,6 | 3,5 |
| 25 g/m² | YI (belichtet 10x10 m/ min) | 6 | 5,8 | 9,2 | 7,6 |
| 25 g/m² | YI (Diff.) | 2,4 | 2,1 | 5,6 | 4,1 |
| | | | | | |
| 100 g/m² | Chem. Test | 1 | 2 | 6 | 7 |
| 10 s | Aceton | 0 | 0 | 0 | 0 |
| 5 h | Rotwein | 3 | 3 | 1,5 | 1 |
| 16 h | Pulverkaffee | 3 | 3 | 2 | 3 |
| 16 h | Schwarzer Johannisbeersaft | 3 | 3 | 0,5 | 1 |
| 10 s | EE-Buac | 0 | 0 | 0 | 0 |
| 5 h | Senf | 3 | 3 | 3 | 3 |
| 16 h | Lippenstift | 2 | 4 | 0 | 4 |
| 10 min | Mercurocrom | 0 | 0 | 0 | 0 |
| 16 h | Schwarze Kugelschreiber-Paste | 4,5 | 4,4 | 4,5 | 4,5 |
| 1 h | Reinigungsmittel | 0 | 0 | 0 | 0 |

### Allgemeine Messmethoden

Viskositäten wurden in einem Rheomat 30 nach DIN 53019 bei einer Temperatur von 23°C und einem Schergefälle D=250 s⁻¹ bestimmt.

Hydroxylzahlen wurden nach DIN ISO 4629 bestimmt.

Säurezahlen wurden nach DIN 53402 bestimmt.

Reaktivitäten wurden folgendermaßen bestimmt. Die Lacke wurden mit einem Spiralrakel in der angegebenen Schichtstärke auf Kunstdruckpapier (120 g/m², halbseitig schwarz bedruckt) appliziert. Die resultierenden Filme wurden auf einem fortlaufenden Band strahlungsgehärtet, und es wurde die Bandgeschwindigkeit ermittelt, bei welcher die Filme am Ende einer vorgegebenen Strecke eine kratzfeste, klebfreie Oberfläche aufwiesen.

Härten wurden anhand der Pendeldämpfung nach DIN 53157 bestimmt.

Flexibilitäten wurden anhand der Erichsen-Tiefung nach DIN 53156 bestimmt.

Der Yellowness Index (YI) ist eine Maß für die Vergilbung. Der YI-Wert wird zunächst direkt nach der Aushärtung (1x10 m/min) und anschließend nach starker Belichtung (10x10 m/min) bestimmt. Je geringer die durch die starke Belichtung verursachte Zunahme des YI-Wertes, desto geringer die Vergilbung.

Die Bestimmung der chemischen Beständigkeit erfolgte nach DIN 68 860 1B. Den in Tabelle 2 angegebene Werten liegt eine Beurteilungsskala von 0 bis 5 zugrunde, wobei 0 für einen nicht angegriffenen Lack und 5 für einen vollständig zerstörten Lack steht.

## Patentansprüche

1. Verfahren zur Herstellung nichtdispergieraktiver urethangruppenhaltiger (Meth)acrylsäureester, wobei man
a) wenigstens eine hydroxylgruppenhaltige Verbindung mit (Meth)acrylsäure in einem Lösungsmittel unter Esterbildung umsetzt;
b) das Lösungsmittel und gegebenenfalls einen Teil nichtumgesetzter (Meth)acrylsäure entfernt;
c) das in Stufe a) *oder* b) resultierende Gemisch mit einer der Säurezahl des Gemisches entsprechenden Menge wenigstens einer epoxidgruppenhaltigen Verbindung umsetzt; und
d) das in Stufe c) resultierende Gemisch mit wenigstens einer isocyanatgruppenhaltigen Verbindung umsetzt.

2. Verfahren nach Anspruch 1, wobei man in Stufe a) wenigstens eine hydroxylgruppenhaltige Verbindung mit zwei oder mehreren Hydroxylgruppen pro Molekül einsetzt, vorzugsweise zwei- bis sechswertige, gegebenenfalls oxalkylierte C₂-C₁₀-Polyole, und/oder Polyesterpolyole aus zwei- bis sechswertigen, gegebenenfalls oxalkylierten C₂-C₁₀-Polyolen und zwei- bis vierwertigen C₃-C₃₆-Carbonsäuren oder veresterbaren Derivaten davon.

3. Verfahren nach Anspruch 1 oder 2, wobei man in Stufe a) gleichzeitig zwei- bis sechswertige, gegebenenfalls oxalkylierte C₂-C₁₀-Polyole mit zwei- bis vierwertigen C₃-C₃₆-Carbonsäuren oder veresterbaren Derivaten davon und (Meth)acrylsäure umsetzt.

4. Verfahren nach einem der vorhergehenden Ansprüche, wobei man in Stufe a) in Bezug auf die Hydroxylgruppen der hydroxylgruppenhaltigen Verbindung(en) einen Überschuss an (Meth)acrylsäure einsetzt, vorzugsweise bis zu einem Äquivalentverhältnis von 1:1,5 und insbesondere in einem Äquivalentverhältnis von 1:1,1 bis 1:1,25.

5. Verfahren nach einem der vorhergehenden Ansprüche, wobei man in Stufe a) als Lösungsmittel einen mit Wasser ein azeotropes Gemisch bildenden Kohlenwasserstoff oder ein mit Wasser ein azeotropes Gemisch bildendes Kohlenwasserstoffgemisch verwendet, vorzugsweise Heptan, Cyclohexan, Methylcyclohexan oder Toluol, oder Gemische davon.

6. Verfahren nach einem der vorhergehenden Ansprüche, wobei man in Stufe b) das Lösungsmittel und gegebenenfalls einen Teil nichtumgesetzter (Meth)acrylsäure destillativ entfernt.

7. Verfahren nach einem der vorhergehenden Ansprüche, wobei man in Stufe c) als epoxidgruppenhaltige Verbindung Polyglycidylether oder -ester polyfunktioneller Alkohole einsetzt, vorzugsweise Bisphenol-A-diglycidylether.

8. Verfahren nach einem der vorhergehenden Ansprüche, wobei das in Stufe c) resultierende Gemisch eine Hydroxylzahl in einem Bereich von 40 bis 150 mg KOH/g aufweist.

9. Verfahren nach einem der vorhergehenden Ansprüche, wobei man in Stufe d) wenigstens eine isocyanatgruppenhaltige Verbindung mit zwei oder mehreren Isocyanatgruppen pro Molekül einsetzt, vorzugsweise Hexamethylendiisocyanat, dessen Isocyanurat, Isophorondiisocyanat oder Gemische davon.

10. Verfahren nach einem der vorhergehenden Ansprüche, wobei man
a) ethoxyliertes Trimethylolpropan und Adipinsäure mit einem Überschuss an (Meth)acrylsäure in einem mit Wasser ein azeotropes Gemisch bildenden Kohlenwasserstoff unter Esterbildung umsetzt;
b) den Kohlenwasserstoff und einen Teil nichtumgesetzter (Meth)acrylsäure destillativ entfernt;
c) das in Stufe b) resultierende Gemisch mit einer der Säurezahl des Gemisches entsprechenden Menge an Bisphenol-A-diglycidylether umsetzt; und
d) das in Stufe c) resultierende Gemisch mit dem Isocyanurat des Hexamethylendiisocyanats umsetzt.

11. Nichtdispergieraktive urethangruppenhaltige (Meth)acrylsäureester, die nach einem Verfahren der Ansprüche 1 bis 10 erhältlich sind.

12. Verwendung nichtdispergieraktiver urethangruppenhaltiger (Meth)acrylsäureester nach Anspruch 11 in strahlungshärtbaren Überzugsmassen.

13. Strahlungshärtbare Überzugsmasse, umfassend wenigstens einen nichtdispergieraktiven urethangruppenhaltigen (Meth)acrylsäureester aus Anspruch 11, wenigstens einen Photoinitiator und gegebenfalls weitere Additive.
